# DEMANDE DE BREVET EUROPEEN

(11) **EP 4 739 004 A2**
(43) Date de publication de la demande: **06.05.2026**
(21) Numéro de dépôt: 26165650.8
(22) Date de dépôt: 09.07.2020
(51) Int. Cl.: H05H 1/24

(54) **PROCÉDÉ DE DÉCONTAMINATION D'UNE PRÉFORME AU MOYEN D'ESPÈCES RÉACTIVES OBTENUES PAR MÉLANGE D'UN AGENT PRÉCURSEUR ET D'UN PLASMA**

(30) Priorité: 11.07.2019 FR 1907781
(62) Demande divisionnaire de: 20736362.3
(71) Demandeur: Sidel Participations, 76930 Octeville-sur-Mer (FR)
(72) Inventeur: LETELLIER, Sandy, 76930 Octeville-sur-Mer (FR); TOUTAIN, Gregory, 76930 Octeville-sur-Mer (FR); SECK, Foune, 76930 Octeville-sur-Mer (FR)
(74) Mandataire: Sidel Group

(57) **Abrégé**

L'invention concerne un procédé de décontamination d'une préforme (14) en matériau thermoplastique par exposition d'au moins une partie de la préforme (14) à des espèces réactives (RS) obtenues par mélange d'un agent (AP) précurseur avec un plasma (P), le plasma (P) étant généré par injection d'un gaz (G) vecteur dans un réacteur (40), caractérisé en ce que le mélange entre l'agent (AP) précurseur et le plasma (P) est réalisé exclusivement en dehors du réacteur (40) avant d'être en contact avec la préforme (14).

L'invention concerne aussi un dispositif (50) de décontamination pour la mise en œuvre du procédé.

## Description

### Domaine technique de l'invention

L'invention se rapporte à un procédé de décontamination d'une préforme en matériau thermoplastique par exposition d'au moins une partie de la préforme à des espèces réactives obtenues par mélange d'un agent précurseur avec un plasma, le plasma étant généré par injection d'un gaz vecteur dans un réacteur.

### Arrière-plan technique

Dans le domaine des emballages agro-alimentaires, il est connu de réaliser un traitement de décontamination pour aseptiser ou stériliser au moins certaines parties d'un récipient destiné à recevoir des denrées alimentaires, notamment l'intérieur et le col du récipient.

Les traitements de décontamination appliqués ont pour but de détruire, ou au moins de réduire, la présence d'organismes microbiologiques ou micro-organismes, tels qu'en particulier les germes, les bactéries, les spores, les moisissures, etc. afin de permettre la conservation des denrées.

Par récipient, on entend un corps creux tel que par exemple une bouteille, un flacon, un pot, etc., qui sont autant de récipients obtenus par transformation d'une préforme en matière thermoplastique qui est le plus souvent préalablement fabriquée par injection dans un moule. La préforme comporte notamment un corps qui est destiné à être conformé à sa forme finale durant un procédé de fabrication, et elle comporte aussi un col qui présente déjà sa forme finale.

Des matières thermoplastiques, le PET (Polyéthylène Téréphtalate) est le plus communément utilisé pour ces applications.

Lors de la fabrication du récipient, le corps de la préforme est tout d'abord conditionnée thermiquement dans un four afin d'en ramollir la matière constitutive. A cet effet, le four comporte des organes de chauffage, notamment par émission de radiations électromagnétiques chauffantes, par exemple par des lampes à infrarouge. La préforme ainsi ramolli est ensuite transformé en un récipient par soufflage dans un moule au moyen d'au moins un fluide sous pression, avec ou sans étirage. Le col de la préforme est maintenu à une température suffisamment froide pour ne pas être déformé durant le procédé de fabrication du récipient.

En variante, la préforme injectée est directement transformée en récipient sans requérir alors de conditionnement thermique préalable.

Dans l'état de la technique, la décontamination d'une préforme est notamment réalisée par "voie chimique" au moyen d'un agent stérilisant comportant du peroxyde d'hydrogène (H₂O₂) ou autre produit présentant des propriétés analogues, c'est-à-dire bactéricide, virucide, fongicide, etc. Il s'agit plus particulièrement d'un agent dit "précurseur" qui est apte à être décomposé en espèces réactives, et notamment en espèces réactives de l'oxygène aussi connues sous leur acronyme anglais "ROS" signifiant "Reactive Oxygen Species". Les espèces réactives de l'oxygène comprennent notamment des radicaux hydroxyles, des anions superoxydes, des ions hydroxyles, etc. Les bactéries et les virus sont très efficacement détruits par contact avec des espèces réactives de l'oxygène.

La décomposition de l'agent précurseur en espèces réactives de l'oxygène est par exemple réalisée par chauffage de l'agent précurseur à température élevée pendant au moins une durée minimale déterminée.

La stérilisation par les espèces réactives de l'oxygène est extrêmement efficace. Elle est en outre particulièrement inoffensive pour les utilisateurs puisque les résidus finaux des réactions chimiques sont constitués uniquement d'eau et de dioxygène.

Ainsi, il est déjà connu d'injecter un agent précurseur, tel qu'une solution de peroxyde d'hydrogène et d'eau, à l'état de vapeur dans une préforme préalablement à son passage dans le four. La vapeur se condense au contact de la paroi interne de la préforme. Ensuite, lors du passage de la préforme dans le four, l'agent précurseur condensé est chauffé en même temps que le corps de la préforme par les organes de chauffage du four. La chaleur est généralement suffisante pour décomposer partiellement l'agent précurseur contenu dans la préforme.

La préforme renferme ainsi une atmosphère stérilisante pendant toute la durée de fabrication du récipient jusqu'au remplissage du récipient.

Cependant, la quantité de chaleur reçue par l'agent précurseur contenu dans les préformes pendant leur chauffage n'est généralement pas suffisante pour décomposer la totalité de l'agent précurseur en espèces réactives de l'oxygène. La quantité de chaleur transmise aux préformes dépend par exemple de la dimension des préformes, de la forme du récipient final, de la couleur des préformes, etc.

Pour résoudre ce problème, il est possible de chauffer les préformes dans le four plus longtemps que nécessaire afin que la plus partie de l'agent précurseur soit décomposée en espèces réactives de l'oxygène.

Néanmoins, il n'est pas toujours possible de chauffer suffisamment l'agent précurseur sans dégrader les préformes.

En outre, la nécessité de continuer à chauffer les préformes alors que leur corps a déjà atteint la température désirée pour pouvoir être conformé en récipient final entraîne une augmentation du coût de fabrication des récipients.

Par ailleurs, si les résidus de réaction des espèces réactives de l'oxygène sont inoffensifs, l'agent précurseur, notamment le peroxyde d'hydrogène, peut être toxique au-delà d'une certaine dose. Ceci peut poser problème, notamment lorsque le récipient est destiné à contenir des produits alimentaires. Pour cette raison, certains pays imposent des normes draconiennes sur la quantité résiduelle d'agent précurseur autorisée dans un récipient après son remplissage. Par exemple, le récipient doit contenir moins de 0,5 ppm d'agent précurseur.

Pour atteindre cet objectif, et en considérant les problèmes de chauffage évoqués précédemment, il est parfois nécessaire de diluer l'agent précurseur dans un solvant inoffensif, par exemple de l'eau, pour diminuer la quantité d'agent précurseur injecté dans le récipient.

Cependant, en diminuant la teneur en agent précurseur, l'efficacité du traitement stérilisant peut être fortement réduite.

Il a déjà été proposé de décomposer l'agent précurseur au moyen d'un plasma. A cet effet, il est connu d'injecter l'agent précurseur mélangé avec un gaz vecteur, tel que de l'air, dans un réacteur de plasma capacitif. Un tel réacteur comporte une chambre de réaction renfermant deux électrodes entre lesquelles une forte différence de potentiel est appliquée. Ceci provoque l'ionisation du gaz vecteur qui active la décomposition de l'agent précurseur en espèces réactives de l'oxygène. Cette solution permet avantageusement de décomposer la quasi-totalité de l'agent précurseur en espèces réactives de l'oxygène avant même d'être mis en contact avec l'objet à décontaminer.

Il est déjà connu que le gaz vecteur se décompose au moins en partie en espèces réactives. Cependant, du fait de la présence de l'agent précurseur, la concentration en espèces réactives est devient extrêmement élevée. La chambre de réaction présente alors une forte concentration en espèces réactives de l'oxygène. Ces éléments, notamment l'ion hydroxyle, ont un très fort pouvoir oxydant et sont par conséquence particulièrement corrosifs. Quel que soit le matériau les composant, les électrodes sont donc rapidement usées et doivent être remplacées très fréquemment.

### Résumé de l'invention

L'invention propose un procédé de décontamination d'une préforme en matériau thermoplastique par exposition d'au moins une partie de la préforme à des espèces réactives obtenues par mélange d'un agent précurseur avec un plasma, le plasma étant généré par injection d'un gaz vecteur dans un réacteur.

Le procédé de l'invention est caractérisé en ce que le mélange entre l'agent précurseur et le plasma est réalisé exclusivement en dehors du réacteur avant d'être en contact avec la préforme. Ainsi, les électrodes du réacteur ne sont jamais en contact avec l'agent précurseur ou avec les espèces réactives obtenues par décomposition de l'agent précurseur au contact du plasma. Selon d'autres caractéristiques du procédé de l'invention :
- au moins une face (28) intérieure de la préforme est exposée aux espèces réactives, les espèces réactives étant confinées à l'intérieur de la préforme pendant une durée déterminée par des moyens de confinement ;
- le procédé est mis en œuvre pendant que la préforme est transportée à travers une installation de fabrication de récipient à partir de préformes comportant un four de chauffage des préformes, les espèces réactives étant injectées dans la préforme avant ou pendant son chauffage dans le four de chauffage, les espèces réactives étant confinées dans la préforme jusqu'à sa sortie du four de chauffage ;
- les espèces réactives sont confinées dans la préforme par un moyen de confinement qui vient se fixer à l'intérieur du col de la préforme.
- les espèces réactives sont confinées dans la préforme par un moyen de confinement qui vient se fixer sur l'extérieur du col de la préforme.

Ce moyen de confinement fixé à l'intérieur ou à l'extérieur du col permet d'isoler l'intérieur de la préforme par rapport à son environnement extérieur.
- les espèces réactives sont confinées dans la préforme au moyen d'un mandrin qui saisit la préforme par son col pour permettre son déplacement dans le four de chauffage ;
- l'agent précurseur est mélangé au plasma à la sortie du réacteur ;
- l'agent précurseur comporte du peroxyde d'hydrogène ;
- l'agent précurseur est à l'état de vapeur lors de son mélange avec le plasma ;
- l'agent précurseur est sous forme de brume lors de son mélange avec le plasma ;
- le gaz vecteur est formé par du dioxygène ;
- le gaz vecteur est de l'air ;
- les espèces réactives comportent un ou plusieurs des éléments chimiques suivants :
   -- les espèces réactives de l'oxygène (ROS) ;
   -- les espèces réactives de l'azote (RNS) ;
   -- l'ozone (O3).

L'invention concerne aussi un dispositif de décontamination pour la mise en œuvre du procédé de décontamination réalisé selon les enseignements de l'invention.

Le dispositif de décontamination est caractérisé en ce qu'il comporte :
- une conduite de décontamination dont une extrémité amont est raccordée à une source d'agent précurseur et dont une extrémité aval est raccordée à au moins une buse d'injection destinée à projeter sur au moins une partie de la préforme les espèces réactives obtenues par décomposition de l'agent précurseur lors de son mélange avec le plasma ;
- un réacteur de plasma qui est raccordé à une source de gaz vecteur et dont une sortie est raccordée à la conduite de décontamination par un embranchement qui divise la conduite de décontamination en un tronçon amont dans lequel est destiné à circuler l'agent précurseur non décomposé, et un tronçon aval dans lequel sont destinées à circuler les espèces réactives de l'oxygène produites par la décomposition de l'agent précurseur lors de son mélange avec le plasma.

Selon d'autres caractéristiques du dispositif de décontamination de l'invention :
- le réacteur de plasma est un réacteur de plasma froid tel qu'un réacteur par décharge Corona ou un réacteur à décharge à barrière diélectrique ;
- la conduite de décontamination comporte un évaporateur de l'agent précurseur qui est agencé en amont de l'embranchement avec le réacteur de plasma ;
- la conduite de décontamination comporte un organe de brumisation de l'agent précurseur qui est agencé en amont de l'embranchement.

### Brève description des figures

D'autres caractéristiques et avantages de l'invention apparaitront au cours de la lecture de la description détaillée qui va suivre pour la compréhension de laquelle on se reportera aux dessins annexés dans lesquels :
[Fig. 1] La figure 1 est une vue schématique de dessus qui représente un exemple d'installation de fabrication de récipients par formage de préformes qui est équipé d'un dispositif de décontamination réalisé selon les enseignements de l'invention ;
[Fig. 2] La figure 2 est une vue de profil qui représente un exemple de préforme susceptible d'être décontaminée par application du procédé réalisé selon les enseignements de l'invention ;
[Fig. 3] La figure 3 est une vue schématique qui représente un dispositif de décontamination conçu pour la mise en œuvre du procédé de décontamination réalisé selon les enseignements de l'invention ;
[Fig. 4] La figure 4 est un schéma-bloc qui représente le procédé de décontamination réalisé selon les enseignements de l'invention ;
[Fig. 5] La figure 5 est une vue en coupe axiale qui représente schématiquement une partie d'une chaîne de convoyage comportant une tournette transportant une préforme à travers le four de chauffage ;
[Fig. 6] La figure 6 est une vue de côté qui représente schématiquement une préforme exposée à un écoulement d'air laminaire qui est apte à confiner des espèces réactives contenues dans la préforme.

### Description détaillée de l'invention

Dans la suite de la description, des éléments présentant une structure identique ou des fonctions analogues seront désignés par une même référence.

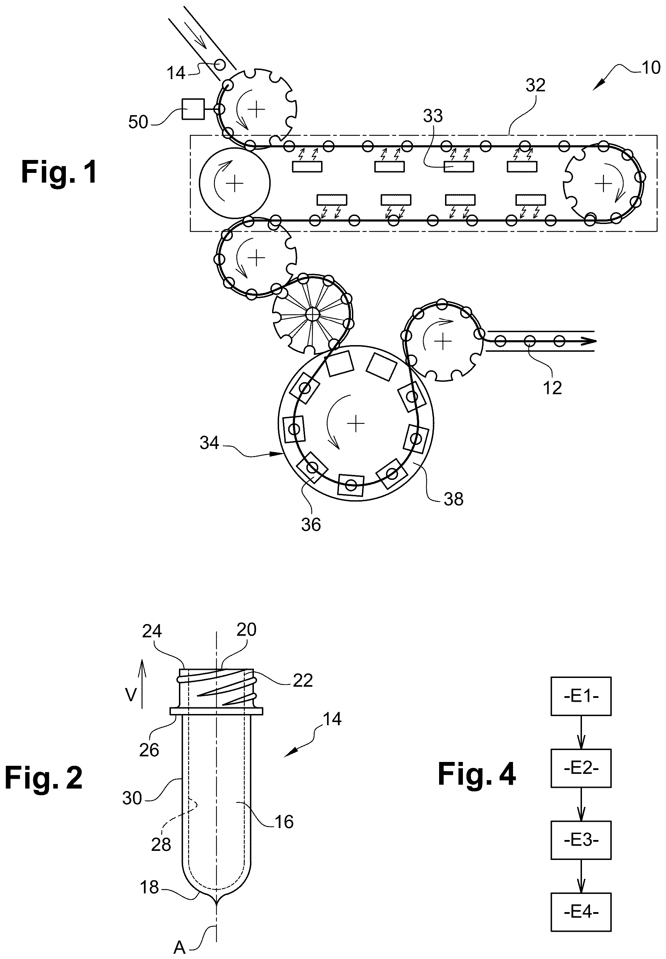

On a représenté à la figure 1 une installation 10 de fabrication de récipients en grande série. Une telle installation 10 permet de fabriquer des récipients 12 finaux par formage de préformes 14. La figure 2 illustre un exemple d'une préforme 14 en matière thermoplastique. La préforme 14 est ici réalisée en polyéthylène téréphtalate (PET).

Une telle préforme 14 est généralement obtenue par injection de matière plastique et présente des caractéristiques (dimensions, répartition de la matière, etc.) qui sont déterminées par le récipient 12 final à obtenir, en particulier sa forme ou encore sa contenance.

La préforme 14 présente une forme axisymétrique d'axe "A" principal représenté verticalement à la figure 2. La préforme 14 comporte notamment un corps 16 tubulaire fermé à une extrémité inférieure par un fond 18 et qui débouche vers le haut par une ouverture 20 supérieure délimitée radialement par un col 22. L'axe "A" principal de la préforme 14 passe au milieu du col 22. Le col 22 de la préforme 14 présente sa forme définitive à l'issue de la fabrication par injection de la préforme 14 et correspond au col du récipient 12 final. Un bord 24 d'extrémité supérieure libre du col 22, aussi appelé buvant, délimite circonférentiellement l'ouverture 20 circulaire constituant le seul accès à l'intérieur de la préforme 14.

Dans l'exemple représenté à la figure 2, le col 22 comporte une collerette 26 qui s'étend radialement en saillie vers l'extérieur.

Le corps 16 est délimité par une paroi tubulaire qui présente une face 28 interne située à l'intérieur de la préforme 14 et représentée en traits interrompus à la figure 2, et une face 30 externe située à l'extérieur.

La préforme 14 présente par exemple une ouverture 20 de diamètre interne d'environ 20 mm et une hauteur totale d'environ 90 mm. L'épaisseur de la paroi délimitant le corps 16 est par exemple d'environ 3 mm. Ces valeurs sont indiquées à titre d'exemple non limitatif, les préformes 14 pouvant présenter des dimensions différentes tout en gardant des proportions globalement identiques.

En se reportant à nouveau à la figure 1, l'installation 10 de fabrication comporte principalement un four 32 de chauffage qui comporte des organes 33 de chauffage du corps 16 des préformes 14 au-delà d'une température de transition vitreuse à laquelle le matériau devient malléable. Pour garantir que le col 22 des préformes 14 ne soit pas déformé durant le procédé de fabrication, le four 32 comporte des moyens pour maintenir la température du col 22 au-dessous de sa température de transition vitreuse.

Le four 32 de chauffage comporte un dispositif 35 de convoyage des préformes 14 en file. Le dispositif 35 de convoyage comporte des supports individuels circulant en circuit fermé dans la station 10 de chauffage.

Dans le mode de réalisation représenté aux figures 2 à 4, le dispositif 35 de convoyage comporte ici une pluralité d'éléments 37 de transport formant les maillons d'une chaîne de transport fermée. Chaque élément 37 de transport porte au moins un support individuel. Chaque support individuel comporte un mandrin 39. Le mandrin 39 est destiné à être emmanché dans le col d'une préforme 14. Selon un exemple de réalisation connu, le mandrin 39 est pourvu de moyens élastiques (non représentés), tel qu'un joint torique, avantageusement réalisé dans un matériau élastique (tel qu'un élastomère), et dont le diamètre externe est égal ou légèrement supérieur au diamètre interne du col d'une préforme 14, de façon à assurer la sustentation de la préforme 14 par frottement contre la paroi interne du col, lorsque le mandrin 39 est emmanché, et d'isoler l'intérieur de la préforme par rapport à son environnement extérieur, garantissant ici la qualité de la décontamination.

Généralement, chaque mandrin 39 comporte des moyens, tels qu'un pignon 41 coopérant avec une courroie fixe (non représentée), susceptibles d'entraîner la préforme 14 en rotation autour de son axe principal durant son déplacement le long d'au moins une partie du trajet de transport pour permettre un chauffage homogène du corps de la préforme 14. Un tel dispositif de transport à mandrin 39 rotatif est parfois appelé "tournette". L'installation 10 de fabrication comporte aussi une station 34 de soufflage qui comporte des unités 36 de moulage apte à recevoir les préformes 14. Les préformes 14 sont par exemple destinées à être conformées en récipient 12 finaux par étirage-soufflage dans un moule (non représenté) d'une unité 36 de moulage. Les unités 36 de moulage sont montés sur un carrousel 38 qui permet de déplacer les préformes 14 durant leur transformation en récipient 12 final depuis un point d'entrée jusqu'à un point de sortie. Durant le procédé de fabrication les préformes 14, puis les récipients 12 sont déplacés en file le long d'un trajet de production déterminé, représenté en trait gras à la figure 1, par des convoyeurs. Les convoyeurs comportent des moyens de support individuels (non représentés) de chaque corps creux à l'état de préforme 14 ou de récipient 12. Ces convoyeurs sont bien connus de l'état de la technique et ne seront pas détaillés ici.

La fabrication des récipients 12 n'est pas réalisée dans une pièce aseptique, même lorsqu'ils sont destinés à un usage alimentaire.

Il est donc nécessaire de prévoir des moyens de décontamination des préformes 14.

L'invention propose un procédé de décontamination d'une préforme 14 en matériau thermoplastique par exposition d'au moins une partie de la préforme 14 à des espèces réactives "RS" obtenues par mélange d'un agent précurseur "AP" avec un plasma "P". Il s'agit plus particulièrement d'espèces réactives qui ont des propriétés germicides. Le procédé de décontamination est avantageusement mis en œuvre pendant la prise en charge des préformes dans l'installation de fabrication de récipients, plus particulièrement pendant le transport des préformes à travers l'installation de fabrication.

Les espèces réactives sont notamment composées d'un ou de plusieurs des éléments chimiques suivants :
- les espèces réactives de l'oxygène, aussi connues sous leur acronyme anglais "ROS" signifiant "Reactive Oxygen Species" parmi lesquelles on trouve le radical hydroxyle, l'anion superoxyde, les ions hydroxyles, le radical perhydroxyl, etc. ;
- les espèces réactives de l'azote, aussi connues sous leur acronyme anglais "RNS signifiant "Reactive Nitrogen Species", parmi lesquelles on trouve notamment le monoxyde d'azote (NO) ;
- l'ozone.

Un tel agent précurseur "AP" est défini comme étant un agent est apte à être décomposé en espèces réactives "RS" par exposition à un plasma "P". Avantageusement, l'agent précurseur "AP" est sélectionné de manière à se décomposer totalement en espèces réactives "RS" de manière qu'il n'y ait pas besoin de nettoyer les récipients 12 à l'issu de leur procédé de fabrication.

Un tel agent précurseur "AP" est par exemple formé par du peroxyde d'hydrogène (H₂O₂), notamment par une solution de peroxyde d'hydrogène et d'eau.

La concentration de peroxyde d'hydrogène dans la solution aqueuse est par exemple comprise entre 5% et 35%. Le peroxyde d'hydrogène et/ou l'eau sont susceptibles de se décomposer très rapidement en de nombreuses espèces réactives de l'oxygène "ROS".

Par ailleurs, les espèces réactives "RS" obtenues par exposition du peroxyde d'hydrogène à un gaz à l'état de plasma "P" sont stables suffisamment longtemps pour permettre de décontaminer les préformes 14 durant tout leur procédé de fabrication. Les réactions entre les différentes espèces réactives "RS" favorisent en effet la promotion de nouvelles espèces réactives "RS".

Le plasma "P" est généré par injection d'un gaz vecteur dans un réacteur 40. Le gaz vecteur est généralement du dioxygène (O₂), en ce cas, les espèces réactives "RS" produites par mélange du plasma "P" avec l'agent précurseur "AP" sont principalement des espèces réactives de l'oxygène.

En variante, le gaz vecteur peut aussi être formé d'un autre gaz pur ou encore d'une composition, par exemple de l'air. Lorsque le gaz vecteur est formé d'un gaz contenant de l'azote, tel que de l'air, le plasma comporte des espèces réactives de l'azote et des espèces réactives de l'oxygène. Ces espèces réactives réagissent avec l'agent précurseur "AP" lors du mélange pour former de nouvelles espèces réactives de l'oxygène. Les espèces réactives issues du mélange comportent donc un mélange d'espèces réactives de l'oxygène et d'espèces réactives de l'azote.

Le réacteur 40 permet ici de générer un plasma "P" froid obtenu à pression atmosphérique. Le plasma "P" est par exemple obtenu par apport d'énergie électromagnétique au gaz vecteur "G". A cet effet, le réacteur 40 est par exemple un réacteur 40 capacitif qui comporte principalement une chambre 42 de réaction dans laquelle se trouvent deux électrodes 44 qui sont raccordées électriquement à un générateur 46 de courant électrique, continu ou alternatif, permettant d'appliquer une forte différence de potentiel entre les deux électrodes 44.

Selon un premier exemple non représenté, il s'agit d'un réacteur à effet corona dans lequel un arc électrique est susceptible de se créer entre les deux électrodes si l'on applique une différence de potentielle supérieure à une tension de claquage entre les deux électrodes.

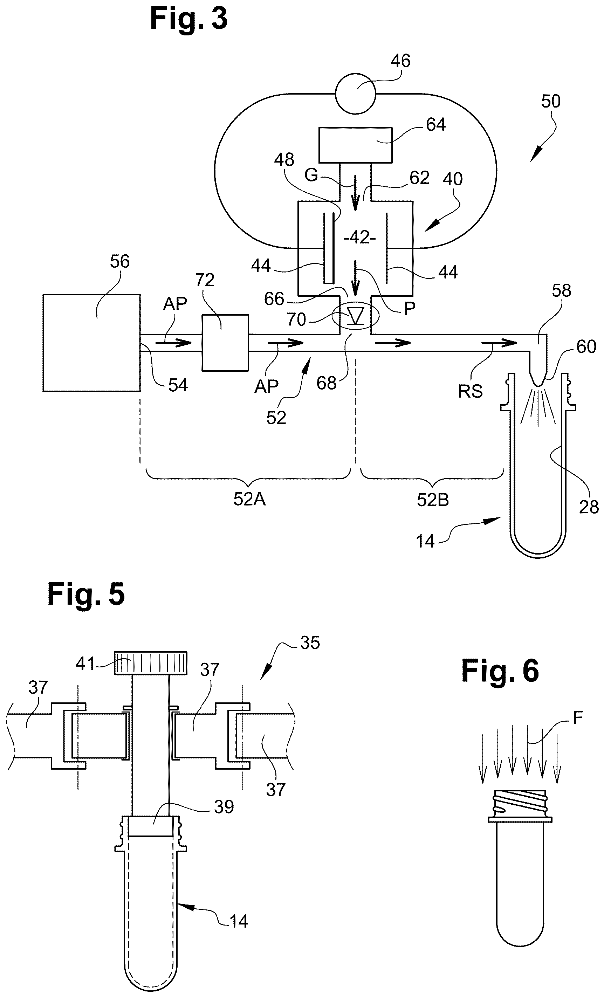

Selon un deuxième exemple représenté à la figure 3, il s'agit d'un réacteur 40 à décharge à barrière diélectrique, ou encore "DBD" selon l'acronyme anglais de "dielectric barrier discharge". Dans ce réacteur 40, une barrière 48 réalisée en un matériau diélectrique est interposée entre les deux électrodes 44. Cela permet notamment d'éviter la production d'arcs électriques entre les deux électrodes 44.

On a constaté que la mise en contact des électrodes 44 avec l'agent précurseur "AP" était susceptible d'user prématurément les électrodes 44 par oxydation.

Pour résoudre ce problème tout en conservant les avantages de la production d'espèces réactives "RS" par plasma "P", l'invention propose que le mélange entre l'agent précurseur "AP" et le plasma "P" soit réalisé exclusivement en dehors de la chambre 42 de réaction du réacteur 40 avant d'être en contact avec la préforme 14. Ainsi, ni l'agent précurseur "AP", ni les espèces réactives "RS" obtenues par contact entre l'agent précurseur "AP" et le plasma "P" n'entrent jamais en contact avec les électrodes 44. Ceci évite ainsi de corroder prématurément les électrodes 44. En outre, le fait de réaliser le mélange avant sa mise en contact avec la préforme 14 permet de décomposer la quasi-totalité de l'agent précurseur "AP". Ainsi, la teneur résiduelle en agent précurseur "AP" dans le récipient 12 réalisé à partir de ladite préforme 14 ainsi traitée est avantageusement négligeable. L'agent précurseur "AP" est avantageusement mélangé au plasma "P" directement à la sortie du réacteur 40. Ainsi, la majorité du gaz vecteur "G" est encore à l'état de plasma "P" lors du mélange avec l'agent précurseur "AP".

Pour favoriser la production d'espèces réactives "RS", l'agent précurseur "AP" est vaporisé préalablement à son mélange avec le plasma "P". Ainsi, l'agent précurseur "AP" est à l'état de vapeur lors de son mélange avec le plasma "P".

En variante, l'agent précurseur "AP" est mélangé au plasma "P" sous forme de brume.

Comme représenté à la figure 4, le procédé de l'invention comporte ainsi une première étape "E1" de production d'un plasma "P" par un réacteur 40, puis une deuxième étape "E2" de mélange de l'agent précurseur "AP" avec le plasma "P" en dehors du réacteur 40, suivie d'une troisième étape "E3" de projection des espèces réactives "RS" obtenues lors de la deuxième étape "E2" sur les parties de la préforme 14 à décontaminer.

Aussi bien les faces 28, 30 intérieures et extérieures de la préforme 14 sont susceptibles d'être décontaminées par ce procédé.

Ce procédé est cependant particulièrement intéressant pour décontaminer la face 28 intérieure de la préforme 14, qui est peu accessible, et son col 22, qui est peu chauffé par le four 32.

Dans le cas de la décontamination de l'intérieur de la préforme 14, le procédé comporte avantageusement, mais pas obligatoirement, une étape "E4" de confinement des espèces réactives "RS" à l'intérieur de la préforme 14 pendant une durée déterminée à compter de leur injection, par exemple moins de 12 secondes.

Lorsque les espèces réactives "RS" sont injectées dans la préforme 14 en amont du four 32 de chauffage, la durée déterminée correspond au temps que met la préforme 14 à sortir du four 32 de chauffage, par exemple moins de 12 secondes. Dans ce cas, le confinement des espèces réactives "RS" est réalisé au moyen du mandrin 39 qui vient boucher la préforme 14 pendant sa traversée du four 32 de chauffage.

Ce confinement permet de s'assurer que les espèces réactives soit localisées à l'endroit où l'on souhaite avoir une décontamination et empêchent ici qu'elles ne s'échappent à l'intérieur du four. La dispersion de ces espèces réactives pourrait occasionner une oxydation de pièces métalliques se trouvant dans le four.

De plus, ce confinement permet de s'assurer que les espèces réactives restent un certain temps en contact avec le volume délimité par la paroi intérieure de la préforme, ce qui garantit l'efficacité de l'étape de décontamination.

Lorsque les espèces réactives "RS" sont injectées dans la préforme 14 en un autre lieu de l'installation, par exemple en aval du four 32 de chauffage, le confinement peut être réalisé par bouchage physique de la préforme 14 au moyen d'un bouchon, ou encore par projection d'un flux "F" d'air laminaire au droit du col parallèlement à l'axe de la préforme 14 directement vers l'intérieur de la préforme 14. Le flux "F" d'air doit alors présenter, en section transversale, des dimensions supérieures à celles du col de la préforme 14 pour éviter que le flux d'air ne chasse les espèces réactives au lieu de les confiner, comme cela est représenté par le faisceau de flèches "F" parallèles à la figure 6. On a représenté à la figure 3 un exemple de dispositif 50 de décontamination pour la mise en œuvre du procédé de décontamination réalisé selon les enseignements de l'invention. Le dispositif 50 de décontamination comporte une conduite 52 de décontamination dont une extrémité 54 amont est raccordée à une source 56 d'agent précurseur "AP" et dont une extrémité 58 aval est raccordée à une buse 60 d'injection qui est destinée à injecter les espèces réactives "RS" sur ou dans une préforme 14. Dans l'exemple représenté, il s'agit d'une buse 60 qui est destinée à injecter les espèces réactives "RS" à l'intérieur de la préforme 14. La source 56 d'agent précurseur "AP" délivre l'agent précurseur "AP" sous pression pour permettre son écoulement vers la buse 60.

Le dispositif 50 de décontamination comporte aussi un réacteur 40 de plasma "P". Le réacteur 40 comporte une chambre 42 de réaction dont au moins un orifice 62 d'entrée est raccordé à une source 64 de gaz vecteur "G" et dont une sortie 66 est raccordée à un tronçon intermédiaire de la conduite 52 de décontamination par un embranchement 68. L'embranchement 68 divise la conduite 52 de décontamination en un tronçon 52A amont dans lequel est destiné à circuler l'agent précurseur "AP" non décomposé, et un tronçon 52B aval dans lequel sont destinées à circuler les espèces réactives "RS" produites par la décomposition de l'agent précurseur "AP" lors de son mélange avec le plasma "P".

Le gaz vecteur "G" délivré par la source 64 est par exemple du dioxygène ou de l'air.

Le gaz vecteur "G" peut être injecté sous pression pour permettre son écoulement vers la sortie 68.

Il est possible de prévoir un clapet anti-retour 70 à la sortie de la chambre 42 de réaction pour éviter que l'agent précurseur "AP" ou les espèces réactives "RS" obtenue par décomposition de l'agent précurseur "AP" ne soient refoulées accidentellement vers la chambre 42 de réaction.

En variante, lorsque le plasma (P) est généré par l'injection du gaz vecteur dans le réacteur, la pression dans le réacteur est supérieure à la pression dans la conduite (52) de décontamination. Par conséquent, pendant la mise en œuvre de ce procédé de décontamination, la pression dans le réacteur étant supérieure à la pression dans la conduite (52) de décontamination, cela évite toute remontée d'agent précurseur (AP) et/ou d'espèces réactives dans le réacteur et permet ainsi d'éviter toute oxydation de ce dernier. Pour ce faire, le plasma, est avantageusement généré avant et après l'étape de vaporisation de l'agent précurseur dans la conduite (52) de décontamination.

La chambre 42 de réaction renferme deux électrodes 44 écartées l'une de l'autre. Les électrodes 44 sont branchées à un générateur 46 de courant électrique. Les électrodes 44 sont agencées de manière que le gaz vecteur "G" circulant depuis l'orifice 62 d'entrée jusqu'à l'orifice 66 de sortie passe dans l'espace ménagé entre les deux électrodes 44. La différence de potentiel appliquée entre les électrodes 44 est suffisante pour transformer le gaz vecteur "G" en plasma "P".

Il s'agit ici d'un réacteur 40 à décharge à barrière 48 diélectrique. Une barrière 48 de matériau diélectrique est interposée entre les deux électrodes 44.

Lorsque l'agent précurseur "AP" est délivré à l'état liquide par la source 56 d'agent précurseur "AP", la conduite de décontamination comporte un évaporateur 72 de l'agent précurseur "AP" qui est agencé en amont de l'embranchement avec le réacteur 40 de plasma "P". L'agent précurseur "AP" est par exemple chauffé au moyen d'une résistance électrique. En variante, il s'agit d'un échangeur de chaleur.

Selon une autre variante de l'invention, l'évaporateur est remplacé par un organe de brumisation de l'agent précurseur "AP" agencé en amont de l'embranchement.

Dans l'exemple représenté à la figure 1, le dispositif 50 de décontamination est agencé pour traiter les préformes 14 en amont du four 32. La chaleur fournie par le four permet en effet d'accélérer les réactions entre les espèces réactives "RS" et les agents microbiologiques présents à la surface des préformes 14. Cependant, il est aussi possible d'agencer le dispositif 50 de décontamination en d'autres endroits de l'installation 10 de fabrication, par exemple en aval du four 32.

On a constaté que la décontamination de l'intérieur de la préforme 14 était encore plus efficace lorsque les espèces réactives "RS" sont confinées à l'intérieur de la préforme 14 après leur injection dans la préforme 14.

Lors de l'utilisation du dispositif 50 de décontamination, le réacteur 40 est alimenté en gaz vecteur "G" pour produire un plasma "P" de ce gaz vecteur "G". Le plasma "P" ainsi obtenu est injecté dans la conduite 52 de décontamination au niveau de l'embranchement 68.

Simultanément, la conduite 52 est alimentée en agent précurseur "AP" qui est vaporisé par le vaporisateur 72. L'agent précurseur "AP" ainsi vaporisé est ensuite mélangé au plasma "P" au niveau de l'embranchement, sans pénétrer dans le réacteur 40. Ce mélange active la décomposition de l'agent précurseur "AP" en espèces réactives "RS". Puis, les espèces réactives "RS" ainsi obtenues sont projetées sur les faces à décontaminer de la préforme 14, ici à l'intérieur de la préforme 14 pour en décontaminer la face 28 intérieur.

Lors de la mise en œuvre de ce procédé, la totalité de l'agent précurseur introduit dans le conduit de décontamination 52 peut être activé en totalité par le plasma.

Dans un mode de réalisation particulier, il est possible qu'une partie seulement de l'agent précurseur introduit dans le conduit de décontamination 52 soit activé par le plasma. Dans ce mode de réalisation, l'agent précurseur est activé de deux manières complémentaires. Il y a une première quantité d'agent précurseur qui est directement activé par le plasma, et la quantité d'agent précurseur non directement activé est injecté dans la préforme. La quantité d'agent précurseur non directement activé est injecté sous forme vapeur dans la préforme et vient alors se condenser sur les parois intérieures du corps de la préforme et sera activé par l'intermédiaire des éléments chauffant, c'est-à-dire, des lampes IR ou des diodes laser.

La décontamination de la préforme par l'agent précurseur, notamment du peroxyde d'hydrogène, activé par chauffage commence lorsque l'agent précurseur atteint une certaine température.
dans ce mode de réalisation ; le procédé de décontamination comprend deux phases d'activation de l'agent précurseur, dont une phase d'activation par plasma permettant une activation de l'agent précurseur quasiment instantané, c'est-à-dire que la décontamination de la préforme commence dès que le mélange activé par le plasma est en contact avec la préforme et notamment le corps de cette dernière. Le procédé une deuxième phase d'activation de la quantité d'agent précurseur non précédemment activée.

Par conséquent, la cinétique des deux phases d'activation sont différentes. La première phase d'activation a une cinétique de réaction plus rapide que la cinétique de réaction de la deuxième phase d'activation

Avantageusement, selon la quantité d'agent précurseur injecté, il est possible de combiner la proportion des deux phases d'activation de l'agent précurseur. Cela permet d'augmenter la cinétique globale de la décontamination de la préforme et donc également son taux de décontamination.

De plus, le fait d'avoir deux phases d'activation permet de gérer la quantité d'agent précurseur résiduel restant dans la préforme à la sortie du four, c'est-à-dire à la fin de la décontamination.

Le procédé selon l'invention et son dispositif 50 de mise en œuvre permettent très avantageusement de décontaminer les préformes 14 à partir d'un agent précurseur "AP" tel que le peroxyde d'hydrogène tout en obtenant un récipient final avec une quantité résiduelle négligeable d'agent précurseur "AP". Il n'est donc pas nécessaire de laver le récipient 12 à l'issu de son procédé de fabrication.

En outre, le fait de mélanger le plasma "P" à l'agent précurseur "AP" permet d'obtenir un résultat aussi satisfaisant qu'en le mélangeant à l'intérieur du réacteur, mais sans provoquer l'usure prématurée des électrodes 44. Ainsi, ce procédé est particulièrement économique car il permet de réduire les interventions de maintenance sur le réacteur 40.

## Revendications

1. Dispositif (50) de décontamination pour la décontamination d'une préforme (14) en matériau thermoplastique par exposition d'au moins une partie de la préforme (14) à des espèces réactives (RS) obtenues par mélange d'un agent (AP) précurseur avec un plasma (P), le plasma (P) étant généré par injection d'un gaz (G) vecteur dans un réacteur (40), comportant :
- une conduite (52) de décontamination dont une extrémité amont est raccordée à une source (56) d'agent (AP) précurseur et dont une extrémité aval est raccordée à au moins une buse (60) d'injection destinée à projeter sur au moins une partie de la préforme (14) les espèces réactives (RS) obtenues par décomposition de l'agent (AP) précurseur lors de son mélange avec le plasma (P) ;
- un réacteur (40) de plasma qui est raccordé à une source (64) de gaz (G) vecteur et dont une sortie (66) est raccordée à la conduite (52) de décontamination par un embranchement (68) qui divise la conduite (52) de décontamination en un tronçon (52A) amont dans lequel est destiné à circuler l'agent (AP) précurseur non décomposé, et un tronçon (52B) aval dans lequel sont destinées à circuler les espèces réactives (RS) produites par la décomposition de l'agent (AP) précurseur lors de son mélange avec le plasma (P),
**caractérisé en ce que** le réacteur (40) est un réacteur (40) capacitif comportant une chambre (42) de réaction dans laquelle se trouvent deux électrodes (44) qui sont raccordées électriquement à un générateur (46) de courant électrique, continu ou alternatif, permettant d'appliquer une forte différence de potentiel entre les deux électrodes (44) et **en ce que** ledit réacteur (40) de plasma est raccordé à une source (64) de gaz (G) vecteur et dont une sortie (66) est raccordée à la conduite (52) de décontamination par un embranchement (68) qui divise la conduite (52) de décontamination en un tronçon (52A) amont dans lequel est destiné à circuler l'agent (AP) précurseur non décomposé, et un tronçon (52B) aval dans lequel sont destinées à circuler les espèces réactives (RS) produites par la décomposition de l'agent (AP) précurseur lors de son mélange avec le plasma (P) de telle sorte que le mélange entre l'agent (AP) précurseur et le plasma (P) est réalisé exclusivement en dehors du réacteur (40) avant d'être en contact avec la préforme (14).

2. Dispositif (50) selon la revendication 1, **caractérisé en ce que** la conduite (52) de décontamination comporte un évaporateur (72) de l'agent (AP) précurseur qui est agencé en amont de l'embranchement (68) avec le réacteur (40) de plasma.

3. Dispositif (50) selon l'une quelconque des revendications 1 ou 2, **caractérisé en ce que** la conduite (52) de décontamination comporte un organe de brumisation de l'agent (AP) précurseur qui est agencé en amont de l'embranchement (68).

4. Dispositif (50) selon l'une quelconque des revendications 1 à 3 **caractérisé en ce qu'**il comporte un clapet anti-retour (70) à la sortie de la chambre (42) de réaction pour éviter que l'agent précurseur "AP" ou les espèces réactives "RS" obtenue par décomposition de l'agent précurseur "AP" ne soient refoulées accidentellement vers la chambre (42) de réaction.

5. Dispositif (50) selon l'une quelconque des revendications 1 à 3 **caractérisé en ce que** la pression dans le réacteur (40) est supérieure à la pression dans la conduite (52) de décontamination afin d'éviter toute remontée d'agent précurseur (AP) et/ou d'espèces réactives dans le réacteur (40) et d'éviter toute oxydation de ce dernier.

6. Dispositif (50) selon l'une quelconque des revendications 1 à 5 **caractérisé en ce qu'**une barrière (48) de matériau diélectrique est interposée entre les deux électrodes (44).

7. Dispositif (50) selon l'une quelconque des revendications 1 à 6 **caractérisé en ce que** la conduite (52) de décontamination comporte un évaporateur (72) de l'agent précurseur "AP" qui est agencé en amont de l'embranchement (68) avec le réacteur (40) de plasma "P".

8. Dispositif (50) selon la revendication 7 **caractérisé en ce que** l'agent précurseur "AP" est chauffé au moyen d'une résistance électrique.

9. Dispositif (50) selon la revendication 7 **caractérisé en ce que** l'agent précurseur "AP" est chauffé au moyen d'un échangeur de chaleur.

10. Dispositif (50) selon l'une quelconque des revendications 1 à 6 **caractérisé en ce que** la conduite (52) de décontamination comporte un organe de brumisation de l'agent précurseur "AP" agencé en amont de l'embranchement (68).
